(19) Europäisches Patentamt

European Patent Office

Office européen des brevets

(11) Publication number: **0 336 655**
**A1**

(12) **EUROPEAN PATENT APPLICATION**

(21) Application number: **89303187.2**

(22) Date of filing: **31.03.89**

(51) Int. Cl.⁴: **G01N 33/68 , G01N 33/574**

(30) Priority: **31.03.88 CS 2191/88**

(43) Date of publication of application:
**11.10.89 Bulletin 89/41**

(84) Designated Contracting States:
**CH DE FR GB IT LI NL SE**

(71) Applicant: **Jednotné zemedelske druzstvo**
**Agrokombinát Slusovice, nositel rádu práce**
**763 15 Slusovice**
**Gottwaldov(CS)**

(72) Inventor: **Bitto, Karol**
**No 2 Kyjevska**
**Trencin(CS)**
Inventor: **Cerhova, Marie**
**No 1678 Slavickova**
**Sokolov(CS)**
Inventor: **Cesal, Milan**
**No 3 Smidkeho**
**Trencin(CS)**
Inventor: **Jansa, Jaroslav**

**No 471 Dostikova**
**Slusovice(CS)**
Inventor: **Klecka, Jiri**
**No 27 Fucikova**
**Plzen(CS)**
Inventor: **Kovarikova, Pavla**
**No 712 Palackeho**
**Chodov(CS)**
Inventor: **Mikan, Antonin**
**No 435 Druzstevni**
**Slusovice(CS)**
Inventor: **Urbanek, Peter**
**No 38 Milose Uhra**
**Trencin(CS)**
Inventor: **Farkas, Petr**
**No 4, 1. maja**
**Trencin(CS)**

(74) Representative: **Arthur, John William et al**
**FITZPATRICKS 4 West Regent Street**
**Glasgow G2 1RS Scotland(GB)**

(54) **Disease detection.**

�减 A technique for screening populations to detect potential patients is based on discovered relationships between acute phase proteins in serum, which relationships are described and exploited by a new cluster analysis method. The individual acute phase proteins used are: C-reactive protein; haptoglobin; alpha-1 acid glycoprotein; and alpha-1 antitrypsin. The technique is especially suitable for the detection of cancer, even when the disease is still in its early stages.

## Disease Detection

The instant invention provides a simple and reliable method for the detection of different diseases, especially cancer and inflammations. The invention also provides the means by which high-risk sections of the population can be screened for early stage cancer.

The main goal of this technique is the accurate selection of the appropriate acute phase proteins and their parallel evaluation by a special cluster analysis method. The following acute phase proteins have been found suitable for the detection of cancer:

C-reactive protein; alpha-1 acid glycoprotein; alpha-1 antitrypsin; and haptoglobin. However the technique is not limited to these specific substances.

Immunoassay techniques may be utilised to detect the levels of acute phase proteins. Vertical turbidimetry and laser nephelometry are both highly suitable for this purpose. From the acute phase protein concentrations in persons with a known diagnosis, it is possible to calculate the clusters for different categories of patients.

Depending on the levels of acute phase proteins in this blood sample, the patient is assigned, according to his state of health, into the appropriate diagnostic group.

The importance of glycoproteins for the determination of inflammatory reactions of the organism has been investigated for many years. Since its introduction in 1937, Brdicka's filtrate has been used for diagnostic purposes without any real understanding of its direct chemical principle. Just ten year later, Winzler et al [R.J.Winzler, I.M.Smyth: Studies on the Mucoproteins of Human Plasma II. Plasma Mucoprotein Levels in Cancer Patients. J. Clin. Invest. 27 (1948) 617] discovered that an increase in the quantity of glycoproteins in blood serum is accompanied by an increase in polarographical current. Homolka [J.Homolka: Polarografie v klinicke chemii. Biochem. Clin. Bohemoslov. 6 (1977) 227] and Kosek [M.Kosek: Biochem. Clin. Bohemoslov. 15 (1986) 43] investigated the diagnostic importance of polarographical activity in human blood serum. Brdicka's reaction was defined as the generation of the so-called protein dual wave in buffer solutions of $Co^{2+}$ or $Co^{3+}$ salts. Its principle is catalysis of hydrogen generation through the effect of the sulphohydril and disulphidic groups of glycoproteins, and also of other serum proteins.

The problem discussed most frequently was the correlation between the filtrate reaction and the diagnosis of tumours' diagnosis [R.J.Winzler, A.W.Devor, J.W.Mehl, and I.M.Smyth: Studies on the Mucoproteins of Human Blood Plasma I, Determination and Isolation. J. Clin. Invest. 27 (1948) 609. M.R.Shetlar: The Serum Polysaccharide Level in Malignancy and in other Pathological Conditions. Cancer Res. 9 (1949) 515]. Most scientists dealing with the problem believe that glycoproteins in serum are increased only where there is tissue degeneration and necrosis and that it is impossible to find any specific changes in blood serum proteins that would enable us to differentiate between malignant and benign processes, including some of the chronic inflammations.

Tumorous disease diagnostics are still rarely based on biochemical methods, which create suspicion rather than confirmation. But in the clinic itself, biochemical experiments cannot be substituted by any other method due to their non-aggressive character and economic advantage, which are important factors in screening vast sections of the population.

Glycoproteins are complexes which are to be widely found in natural sources as most natural proteins contain a glycoprotein component. Biologists are mainly interested in those glycoproteins that are on the surface of the cell, forming part of the cell membrane structure. The importance of these components lies in the fact that glycoproteins probably share responsibility for the determination of cell individuality, serve as antigen determinants, receptors, markers and are symbols of cell identity. It is assumed that the saccharide structures situated on the cell surface form part of the detection mechanism by means of which a cell reacts to the presence of other cells. There is even speculation that this is the principle behind the mechanism controlling tissue growth, and it is believed that the communicative capabilities of tumorous tissues are restricted. The hypothesis has been express [M.A.Harper: Prehled fyziologicke chemie. Avicenum, Praha (1977)] that the glycoproteins of a tumour prevent the host organism from detecting the tumour as a non-self antigen structure.

The increase in glycoproteins in the serum as the organism's reaction to pathological changes has not yet been properly investigated. Grafova et al. [R.Grafova, V.Skulikova, C.Helsich, and C.Sobeslavsky: Zmeny serovych glykoproteinu u plicni rakoviny. Cas. Lek. Ces. 117 (1978) 1422] have also dealt with the application of glycoprotein determination to oncological research. They specialised in examining lung tumours and attempted to discover whether there are other more specific changes apart from the increase in glycoprotein total. They fractionated glycoproteins by means of electrophoretic separation on an acetyl cellulose carrier with a subsequent specific counterstaining of the saccharides after oxidation of the 1.2 hydroxyl groups by periodic acid-Schiff's reagent.

These scientists have found that an increase in glycoproteins is to be seen in all processes related to tissue degeneration and decomposition, even in malignant growth. They point out that there are no significant differences in glycoprotein totals of various stages of tumour growth, but at all stages they were increased. Through fractionation they have discovered significant increase in alpha-1 and alpha-2 fractions and a reduction in beta glycoproteins. They do not consider the changes to be specific. Similar results - an increase in alpha-3 and a reduction in beta glycoproteins factions - have been obtained from people suffering from tuberculous inflammations. The scientists quoted here believe that the monitoring of glycoprotein totals is sufficient for diagnosis purposes and they see its greatest advantage in the evaluation of post-operative progress.

In 1984, Cesal et al. (Czech patent No. 238 020) published a method for the separation of serum glycoproteins by means of DEAE Sephadex 50, (Sephadex is a Trade Mark of Pharmacia AB, Uppsala, Sweden) i.e. by a combination of molecular filtration and ion exchange chromatography. On the basis of a comparison between a group of people suffering from acute and chronic inflammations and tumours and a group of healthy people, the conclusion was drawn that serum glycoproteins can be separated into several fractions by means of sodium chloride in various concentrations. The determination of glycoprotein concentrations in two fractions taken together offers greater diagnostic possibilities than a mere determination of glycoprotein totals.

All the abovementioned methods have, however, very little estimative capability and cannot be used for the direct diagnosis of malignant growth.

No concrete procedure for evaluating the biochemical data obtained using them has been defined and no certain solutions have been found. Their sensitivity and selectivity amounts to some 50% - 60%.

Remarkable progress has been achieved by Cesal, Jansa et al. (Czech patent No. 262 663) who have introduced a new method based on the separation of serum glycoproteins, where the evaluation of glycoprotein concentrations in fractions is carried out by means of an original cluster analysis method. In most cases, the sensitivity and selectivity of the method is 80%, regardless of the tumour type. But in view of the time-consuming separation procedure, the large volume of blood samples and skills required for proper determinations, this method is not suitable for screening purposes, but it can be used more like a clinical reference method.

As the separation on DEAE Sephadex columns is laborious and time-consuming, a more sophisticated method of analysis was looked for. Direct immunoprecipitation methods are widely used for serum protein assays. These can be easily automated and are, furthermore, methods that can be performed in any laboratory and with minimal equipment. Therefore, the serum proteins were separated on a DEAE Sephadex A 50 Pharmacia preparative column. The column was 35 cm in length and 15 mm in diameter. Before the column was filled, Sephadex was allowed to swell in basic TRIS-HCl buffer of 7.0 pH, concentration of 50 mmol/1, for 24 hours. After filling the column, it was washed through with 300 ml basic buffer. After stabilisation of the column, 1.5 ml serum was separated off containing about 0.1 g serum protein. After the serum had penetrated the material in the column, it was washed down with 2 ml basic buffer and then gradient elution was applied.

The starting point of the elution gradient was 300 ml basic TRIS-HCl buffer of 7.0 pH and 50 mmol/1 concentration. The solution was linearly mixed with 300 ml of the same buffer with additional Cl- to achieve final concentration of 500 mmol/l. The rate of flow was 30 ml per hour and fractions of 2.5 ml volume were collected.

The distribution of individual types of serum proteins along the gradient was verified by separating them in agarose or polyacrylamide gel. According to the protein concentration we either used a native fraction of the eluate or an eluate concentration from an Amicon separator. Having obtained a basic orientation, protein detection was performed by immunophoresis and by immunoprecipitation with polyvalent and monovalent antisera. Quantitative determination of individual glycoproteins was carried out according to Mancini [G.Mancini, A.O.Carbonara, and J.S.Heremans: Immunochemical Quantitation of Anginenens by Single Radial Immunodiffusion. Immunochemistry 2 (1965) 235]. The immunoprecipitation process was one commonly used.

Alpha-1 antitrypsin (A1AT), alpha-1 acid glycoprotein (A1AG), and haptoglobin (HpG) were discovered to be the major components of acido-resistant fraction of serum used for separation and afterwards for polarography and photometry. Thus the direct determination of these three glycoproteins can substitute for separation, precipitation and polarography or photometry.

The introduction of another parameter dealing with protein metabolism was considered for its possible contribution to distinguishing between inflammations and tumours. Therefore the C-reactive protein (CRP) was added to the abovementioned glycoproteins. It was found that healthy people have 0.002 +- 0.001 CRP 1, patients suffering from inflammation 0.067 +- 0.045 g/1, and patients with tumours 0.024 +- 0.015 g/l. It is clear that the CRP level is significantly increased in the groups of patients suffering from inflammations and tumours, the increase being not so pronounced in the tumour group. Normally CRP is a blood component which is present in a very low concentration. But its quantity is increased with tissue damage, tumours and inflammations of various origin. CRP is a sensitive factor for the momentaneous assessment of inflammatory processes since it interferes with the partial mechanisms of non-specific immunity. A less significant CRP increase with tumours as compared to a much more significant increase with inflammations is very useful for differential diagnosis. There is quite a significant overlapping of these groups which is illustrated by the high standard deviations. CRP alone is not able to serve as distinguishing parameter and only a parallel evaluation of at least two acute phase proteins (CRP is considered to be one) can achieve this.

The advantages of a multi-dimensional processing of values over a one-dimensional analysis can be briefly summarized as follows. Each sample is represented by a set of four coordinates (w, x, y and z, concentrations of the abovementioned acute phase proteins A1AG, A1AT, HpG and CRP) in a two- to four-dimensional space. Having collected together enough samples from patients whose state of health is already known (normal, suffering from inflammation, or from tumour), we find that clusters for the groups separate out to a significant degree within this space. The centres of these clusters and their covarient matrices may be mathematically described, thereby calibrating the space for individual states of health. Using both well-established and newly-developed statistic calculations and discriminatory analysis we may then calculated the probability (or the proportional quantity) with which we can classify a subject whose diagnosis has not yet been made into one or another group. The diagnosis is consequently made according to the cluster to which the subject most probably belongs. This procedure is preferably carried out using the program "CLUSTEX" created for the parallel evaluation of biochemical parameters.

"CLUSTEX" is a program which aims to diagnose inflammations and tumorous diseases effectively, and is designed for use mainly, but not exclusively, in this field. The diagnostic process used in the program is founded on a fresh analytic approach deduced from knowledge which is already available. Each patient examined is put into one of three categories. This classification depends upon the selected biochemical parameters to which the sample given by the patient corresponds. The program then analyses the biochemical parameters relevant to that group and suggests a diagnosis.

Where standard discrimination analysis is irrelevant the program employs a unique set of discrimination analysis calculations. A basis version of the program is available with prewritten data files which allows a skilled practitioner to make a diagnosis on the basis of the polarographic analysis of blood serum mucoproteins and on the photometric analysis of acute phase reactants. The multiplicity of biochemical methodologies makes it difficult to achieve the kind of standardisation which ensure uniform results from different working places. Therefore, the option of modifying or preparing the basis data on the groups of donors at every workplace where the method will be used has been included. For this reason the program includes procedures for the preparation of "basic methodology files" containing data on the groups of donors. These procedures also offer the possibility of creating new and more successful or simpler methods which allow the skilled practitioner to carry out the differential diagnosis of particular groups of diseases quickly and cheaply.

It is clear from the above statements that the program fulfils many functions and may be applied to fields other than differential diagnostics.

The program may be applied in the following areas of medical practice:

a) The differential diagnosis of groups of diseases.

b) The screening of the healthy population with the object of using differential diagnostics to pick up inflammations and tumours while still in the early stages of development.

c) The monitoring of the progress of the disease and the success of the treatment.

An area, for example, the population of this country, must first be selected in which to carry out the observations (for example, measuring a series of biochemical characteristics). The area is then divided into subsets which cover the whole area continuously. For the differential diagnosis of tumours and inflammations this might be the division of the area into three groups: healthy subjects; patients with inflammations; and patients with tumours. From these subsets a group of people, which for convenience shall be referred to as groups of donors, are selected. Provided that the biochemical characteristics under observation is appropriate, it will be seen that the different donor categories show different average values. If two biochemical characteristics are selected for observation and the results drawn onto a two-dimensional diagram, the donor categories will form partially overlapping groups of points which shall be called "clusters". This process can be extended to include three or more observed biochemical characteristics.

The program cannot only process data on the donor groups, but also create main methodology files which are stored under the file name *.CLS, where * stands for the name of the file. It can create, in addition, shortened versions of a main (basic) file which can similarly be stored as *.CLS.

Information on the methodology currently in use is held in the data file "CLUSTERS". When the program is running, the CLUSTERS file is automatically loaded into the main memory and the program's diagnostic procedures can be immediately initiated. The CLUSTERS file can be modified by the SetUp procedure which creates new file clusters from selected methodology *.CLS files.

The sensitivity of the decision-making algorithm in the clusters interface is very important to the classification (diagnosing) of the subject under observation (the patient) into a particular cluster, as this is the area where most of the clinically uncertain cases will occur. An unsuitable method can cause the diagnosis to fall wide of the mark. CLUSTEX helps the skilled practitioner to classify the subject into one of the clusters and simultaneously calculates the probability with which the patient has been classified into this particular cluster (fuzzy approach).

CLUSTEX can deal with the classification of up to ten separate groups based on the analysis of up to ten observed characteristics.

To maintain the maximum diagnostic efficiency of the program the following conditions must be set:-

1. It must be ensured that the donors are as representative as possible of their category.

2. The normality of the result of measuring the distribution within the donor groups must be ensured.

3. The donors in a particular group must number at least several of tens.

4. The conjunction of all subsets of the space into which the subjects are to be classified has to be whole observed space.

5. The intersection of any two subsets of the space into which the objects are to be classified has to be an empty space.

Given the nature of biochemical data it is practically impossible to fulfil all these conditions, but the fulfilment of conditions 1. and 3. may be considered as being essential. If the other conditions are not met the diagnostic efficiency will be decreased. Nevertheless, practical experience has so far shown that the diagnostic efficiency is still very high, certainly an improvement on other methods.

Three or four of these characteristics are preferably chosen on which to base a diagnosis.

The invention will now be further described by way of Example.

EXAMPLE 1

1. Basic TRIS-HCl buffer: 50 mmol/l aqueous solution whose pH has been brought to pH 7.0 with concentrated hydrochloric acid HCl.

2. Eluting solution A: as much NaCl is weighed into the basic buffer as to obtain a resulting $Cl^-$ concentration of 45 - 50 mmol/l.

3. Eluting solution B: as much NaCl is weighed into the basic buffer as to obtain a resulting $C^-$ concentration of 320 - 330 mmol/l.

4. Eluting solution C: as much NaCl is weighed into the basic buffer as to obtain a resulting $Cl^-$ concentration of 480 - 500 mmol/l.

5. DEAE Sephadex A-50 gel ion exchanger: cca 1.0 g native substance is embedded by 100 ml basic buffer. The buffer forms free liquid on top of the gel layer. Gel suspension must be prepared 24 hours in advance and has a life of two months. It must be kept refrigerated.

6. 2 N ammonia

7. Stock solution of cobalt hexaammotrichloride: 0.267 g $Co(NH_3)6$ Cl6 and 5.35 g $NH_4Cl$ are dissolved in water. Distilled water is added to make up 500 ml.

8. Brdicka trivalent cobalt solution: solutions 4 and 5 are mixed. The life-time of the solution is about one month.

9. 20% aqueous solution of sulphosalicyclic acid.

10. 0.1 N KOH aqueous solution.

Separation Procedure

3.5 ml gel is put into a plastic column (Made by BIORAD Co. for porphyrin determination). The quantity of gel to be put into the column is marked in advance on the column wall. After the last drop from the sintered glass, about 2 ml eluting solution A is pipetted onto the surface of the gel with great caution and allowed to soak into the gel. A beaker marked "eluate A" is put under the column. First only 1 ml of eluting solution A is pipetted with caution onto the surface of the gel layer which has been steeped in serum and is allowed to flow through (making sure not to stir the gel). Then a further 3 ml of eluting solution A is pipetted in. After the last drop, eluate A is obtained. Eluting solution A washes out mostly beta and gamma globulins. This eluate is not used for further analysis. It merely serves to selectively prepare eluates B and C. A beaker marked "eluate B" is then put under the column. Onto the gel surface is pipetted 1 ml eluting solution B which is allowed to flow through. This is followed by a further 2 ml of the same. After the last drop eluate B is obtained. It should be stirred thoroughly. Finally a beaker marked "eluate C" is put under the column and 2 ml of eluting solution C is pipetted onto the gel surface. After the last drop eluate C is obtained. It must also be stirred well. The optimum quantities of eluting solutions have been determined empirically.

Next steps:

0.4 ml of the serum to be examined is put into a test tube marked "S". 0.6 ml eluate B is put into a test tube marked "E300" and 0.6 ml eluate C is put into a test tube marked "E500".

0.1 ml 0.1 N KOH is then added to each of the three test tubes, the contents stirred and allowed to stand for 45 minutes. 1.0 ml 20% sulphosalicyclic acid is then added in drop by drop, stirred well and kept standing for ten minutes. The precipitates are then filtered through FILTRAK 391 or FILTRAK 1390 filter paper. The eluate filtrate must be clear. If it is not it must be centrifuged at 5,500 -6,000 r.p.m. for ten minutes in a cooler centrifuge at 2°C, if possible. Then 0.2 ml filtrate is pipetted from test tube S, 0.8 ml filtrate from the test tubes E300 and E500. The E300 eluate filtrate must be clear, this is a condition for its further processing and an evaluation of the result. If the contents of test tubes E300 and E500 were centrifuged, the required quantity of filtrate must be sucked off with caution so as not to disturb the settled proteins. The optimum quantities of the filtrates have again been determined empirically. Finally 2 ml Brdicka solution is added to each test tube and polarography may start.

Polarography is carried out within the range of 0.6V -cca 1.6 V (creation of polarographical protein dual wave). The voltage is increased by 400 mV/min. This is cathode polarisation, and the anode may be formed by just a silver wire. Polarography may be carried out directly in the particular test tubes.

From wave height obtained on the examined material we deduct the wave height of the blank test performed on Brdicka trivalent cobalt solution within the range of 1.3   -1.45V. The resulting values are subject to further mathematical treatment which leads eventually to a diagnosis.

EXAMPLE 2

Solutions

1. Basic TRIS-HCl buffer: 50 mmol/l aqueous solution whose pH has been brought to pH 7.0 with concentrated hydrochloric acid HCl.

2. Eluting solution A: as much NaCl is weighed into the basic buffer as to obtain a resulting $Cl^-$ concentration of 45 - 50 mmol/l.

3. Eluting solution B: as much NaCl is weighed into the basic buffer as to obtain a resulting $Cl^-$ concentration of 320 - 330 mmol/l.

4. Eluting solution C: as much NaCl is weighed into the basic buffer as to obtain a resulting $Cl^-$ concentration of 480 - 500 mmol/l.

5. DEAE Sephadex A-50 gel ion exchanger: cca 1.0g native substance is embedded by 100 ml basic buffer. The buffer forms free liquid on top of the gel layer. Gel suspension must be prepared 24 hours in advance and has a life of two months. It must be kept refrigerated.

6. Physiological solution.

7. 1.8 mmol/l perchloric acid.

8. 5% wolframophosphoric acid in 2N HCl.

9. Phenol reagent by Folin-Ciocalteau diluted before use with distilled water in a ratio of 1:2 (procedure worked out by Hmolka: linicke bioch.vysetr.metody, SZN, 1969, page 399).

10. 1/5 saturated $Na_2CO_3$ solution: saturated $Na_2CO_3$ solution is prepared first, then it is filtered and diluted with distilled water in a ratio of 1:4.

11. Stock standard solution: 20 mg tyrosin is dissolved, 1/5 saturated $Na_2CO_3$ solution is added to make up 100 ml.

12. Standard solution: to 1 ml stock standard solution is added 1/5 saturated $Na_2CO_3$ solution to make up 10 ml.

Procedure

The separation of serum fractions is the same as in Example 1, resulting in eluates B and C.

Further Procedure

0.25 ml of the serum to be examined is pipetted into a test tube marked "S". 1.5 ml eluate B and C are pipetted into test tubes marked B and C. 2.25 ml physiological solution is added to test tube S and 1.0 ml into test tubes B and C. The contents of the three test tubes are stirred thoroughly and 1.25 ml 1.8 mol/l HC104 is added to each. The contents are stirred once more, left to stand for five minutes and then centrifuged for ten minutes at 5,000 r.p.m. 2.5 ml supernatant is pipetted into a further set of test tubes marked "S", "B" and "C". The supernatant is to be pipetted very carefully so as not to disturb the glycoprotein sediment. 0.5 ml 5% solution of wolframophosphoric acid is added, stirred thoroughly, left standing for five minutes and centrifuged at 5,000 r.p.m. for ten minutes. Then the supernatant is decanted, the test tubes inverted, and the rims dried with filter paper. 3.25 ml 1/5 saturated $Na_2CO_3$ solution is added to each of the test tubes and stirred thoroughly until the glycoprotein precipitate dissolves. Finally 0.5 Folin-Cioc. reagent is added to each test tube and the contents stirred thoroughly.

Blank.Test

3.25 ml 1/5 saturated $Na_2CO_3$ solution is mixed with 0.5 ml Folin-Cioc. reagent.

Processing of Standard Solution (see sub.12)

0.5 ml standard solution is mixed with 2.75 ml 1/5 saturated $Na_2CO_3$ solution and 0.5 Folin-Cioc. reagent. All the test tubes must be stirred thoroughly (test, blank, standard) and then must be kept for forty-five minutes in the dark.

Photometry

After forty-five minutes the absorbance of S, B and C solutions and standard is measured against the blank test at 600 mm in a 1 cm cuvette. The resulting values are subject to further mathematical treatment which eventually leads to a diagnosis.

EXAMPLE 3

As a suitable method of processing a large series of samples to determine the acute phase proteins, vertical turbidimetry on microplates is second only to automated analysis.

The solutions

- Buffer pH 7.2 - 7.4: 7.0 g NaCl
0.2 g $KH_2PO_4$
2.9 g KCl

7

1.0 g NaN$_3$ dissolved in 1 l distilled water
- Turbidimetric medium: 5% w/v polyethylene glycol 6000 in buffer
- Human control serum with declared levels of A1AG, A1AT and HpG
- Standard CRP
- antisera against human A1AG, A1AT, HpG and CRP

Preparing the reagents

- Diluted antiserum: it is necessary to leave the antiserum to dilute in the turbidimetric medium for at least one hour, a whole day for preference, before use. If it is cloudy it can be filtered or put in a centrifuge. The diluted antiserum can be stored in a refrigerator at a temperature of between 2C - 8C where it will keep for several weeks. All the antisera are diluted in the turbidimetric medium to a ratio of 1:4 v/v, except for antiserum A1AT which is diluted to a ratio of 1:2.
- Diluted turbidimetric medium: the buffer is added to the turbidimetric medium in the same ratio as was the antisera, i.e. 1:4, or 1:2.

The determination process

NOTE: all solutions must be brought to room temperature before use.

The volume and concentration of the samples, and the range of the calibration curve is given as follows:-

| protein | declared concentration (g/l) | dil.range of calib. curve (times) | dilution of sample (times) | sample volume (ul) |
|---|---|---|---|---|
| AIAG | 0.7 | 4 - 64 | 41 | 50 |
| AIAT | 2.2 | 8 - 128 | 41 | 50 |
| HpG | 1.7 | 8 - 128 | 41 | 50 |
| CRP | 0.093 | 1 - 64 | 1 | 20 |

The calibration curve:

Prepare a series of serum dilutions from the control serum (2,4,8,16,32,64 and 128) in the following way:-
1. pour 0.5 ml of buffer solution into seven test tubes.
2. to the first test tube add 0.5 ml of the control serum and mix thoroughly.
3. pour 0.5 ml from the first test tube into the second and again mix thoroughly.
4. continue this pattern until you have diluted the control serum by 128 times. This series of concentrations serves to determine the levels of A1AG, A1AT and HpG. To determine the CRP, a special series of dilutions must be prepared from a standard containing a high level of CRP.

The dilution of the samples:

The CRP is determined directly from the undiluted sera, whilst for the remaining three glycoproteins it is diluted with the buffer in a ratio of 1:40 (41 parts in all) i.e. 0.05 ml of serum to 2 ml of buffer.

The blind experiment (blanks):

1. The reference blank (superblank) serves to set the zero value in the reader. It is prepared by pipetting 20 ul or 50 ul of buffer (for CRP, and A1AG, A1AT, HpG respectively) and 200 ul of diluted turbidimetric medium.

2. The antibody blank is prepared from 20 ul (50 ul) of buffer and 200 ul of diluted antiserum.

3. Sample blanks are only necessary for CRP and consist of 20 ul of undiluted sample to 200 ul of diluted turbidimetric medium. The absorbance of sera that has been diluted 41 times is so low that it can be left out of the determination process for A1AG, A1AT and HpG.

4. The standard blank is only prepared for the highest concentration as you may use it to calculate the subsequent dilution blanks. 20 ul (50 ul) of the highest standard concentration is mixed with 200 ul of diluted turbidimetric medium.

## Method:

The layout of standards, blanks and samples on a microplate:

1A - Superblank

1B - Blank for the highest concentration standard (any further standard blanks will finally be placed in the first column)

2A - Antisera blank

2B-H - Calibration curve

3,5,7,9,11 - Sample blanks (if any)

4,6,8,10,12 - Samples

The user may change this layout accoridng to his needs.

Whilst working in replicate, parallel samples are placed in adjacent columns.

Pipette 20 ul or 50 ul of buffer into wells 1A and 2A.

Pipette 20 ul or 50 ul of the most concentrated standard into wells 1B and 2B, and continue this process for the remaining diluting of the standard, pipetting them into column 2.

Pipette 20 ul or 50 ul of the samples into columns 3 to 12. Should it also be necessary to determine the blanks, the same solution must be pipetted into wells 3A and 4A, 3B and 4B, and so on.

Pipette 200 ul of the diluted turbidimetric medium into column 1, and should one wish to determine the sample blanks, also pipette into columns 3, 5, 7, 9 and 11.

Pipette 200 ul of the diluted antisera into columns 2, 4, 6, 8, 10 and 12. If there are no sample blanks one may also use columns 3, 5, 7, 9 and 11.

Keep at room temperature for thirty minutes and prick out the bubbles that appear on the surface before measuring. The wavelength to use when measuring the density is 340 nm.

## The calculation process

The calibration is set by calculating the concentration at each individual point along the curve using the equation:

$$c(i) = p * d / n(i)$$

where:

c (i) is the concentration at a given point;

p is the declared concentration of the protein in the standard human serum;

d is the sample dilution (41 or 1);

n (i) is the standard serum dilution at the point in question (1,2,4,8,16,32,64,128)

The antisera blanks are subtracted from all the values that have been measured and the standard blank and its fractions are subtracted from the respective points on the dose-response curve. Apart from this, when determining the CRP the sample blanks are substrated from the samples. The net density will appear on the dose-response curve from which the value of the samples may be deduced.

From the measurements set forth in all the examples described above it is possible to determine the concentrations of the acute phase proteins.

These concentrations are then subject to parallel evaluation by the cluster analysis method which has been developed expressly for the purpose of evaluating biochemical "fuzzy" data. This method classifies donors into groups according to their state of health.

The invention may be applied to other diseases by using different parameters.

**Claims**

1. An improved method for the determination of the mucoprotein vector of blood serum wherein the concentration of C-reactive protein and one, two or three of the glycoproteins haptoglobin, alpha-1 acid glycoprotein and alpha-1 antitrypsin is determined by precipitation with monospecific antibodies, each resulting concentration value then being plotted on axes w, x, y and z, giving a point which defines the mucoprotein vector characteristic of the blood sample in question.

2. A method according to claim 1 wherein the concentration of C-reactive protein, alpha-1 acid glycoprotein and alpha-1 antitrypsin in blood serum is determined.

3. A method according to claim 1 wherein the concentration of C-reactive protein and alpha-1 acid glycoprotein in blood serum is determined.

4. A method according to claim 1, 2 or 3 wherein the concentration of the proteins is determined by vertical turbidimetry after precipitation.

5. A method for screening a patient for presence of a disease, said method comprising the steps of:

   a) determining the concentrations of chosen acute phase proteins in a blood serum sample

   b) creating clusters from the sets of results taken from patients belonging to groups, the members of which have a well defined diagnosis

   c) evaluating in parallel the results obtained from the serum of an undiagnosed patient by the method of cluster analysis referred to hereinbefore, either enabling the patient to be classified into some of the groups used in the creation of the clusters or leaving him unclassified.

6. A method according to claim 5 wherein the acute phase proteins are: haptoglobin; alpha-1-acid glycoprotein; alpha-1-antitrypsin; and C-reactive protein.

7. A method according to claim 6 wherein the determination of the acute phase proteins comprises a method of involving immunoassay techniques.

8. A method according to claim 7 wherein the assaying of the sample is performed by a method comprising the following steps:

   a) incubating a part of the said sample with an antiserum specific to the given acute phase protein;

   b) measuring the precipitate thus obtained by vertical turbidimetry

   c) calculating the concentration of the chosen acute phase protein using a dose-response curve

9. A method according to claim 5 wherein, rather than measuring the concentrations of the acute phase proteins directly, the total acute phase proteins concentrations in defined fractions of serum are measured.

10. A method according to claim 9 wherein the measurements are carried out by photometry.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 447 545  (S.R. DEFAZIO et al.)<br>* Column 2, line 57 - column 3, line 43; column 6, line 44 - column 8, line 21 *<br>--- | 1,5,7 | G 01 N   33/68<br>G 01 N   33/574 |
| X | EP-A-0 250 216  (JEDNOTNE ZEMEDELSKE DRUZSTVO AGROKOMBINAT SLUSOVICE)<br>* Whole document * | 1,5,9,10 | |
| Y | | 1-10 | |
| | --- | | |
| Y | CHEMICAL ABSTRACTS, vol. 107, 1987, page 328, abstract no. 112236r, Columbus, Ohio, US; & JP-A-61 283 868 (TEIJIN LTD) 13-12-1986<br>* Abstract *<br>--- | 1-10 | |
| X | BIOLOGICAL ABSTRACTS, vol. 32, Philadelphia, PA, US; K. BITTO et al.: "Canscreen early stage diagnostics of cancer by means of mucoprotein vector", & 1986. Vol 0, no 0 p1308<br>--- | 1,5 | |
| Y | CHEMICAL ABSTRACTS, vol. 107, 1987, page 542, abstract no. 37945r, Columbus, Ohio, US; EP-A-199 196 (KURARAY CO. LTD) 29-10-1986<br>* Abstract *<br>---                         -/- | 1-4,6-10 | TECHNICAL FIELDS SEARCHED (Int. Cl.4)<br><br>G 01 N |

The present search report has been drawn up for all claims

| Place of search | Date of completion of the search | Examiner |
|---|---|---|
| THE HAGUE | 17-07-1989 | VAN BOHEMEN C.G. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | CHEMICAL ABSTRACTS, vol. 103, 1985, page 536, abstract no. 176680f, Columbus, Ohio, US; A.M. KNAPP et al.: "Determination of C-reactive protein using turbidimetry. Adaptation to the sebia F.P-901 analyzer. Comparison with the Beckman ICS kinetic method", & FEUILL. BIOL. 1985, 26(146), 51-4 * Abstract * | 1-4,6-10 | |
| Y | CHEMICAL ABSTRACTS, vol. 100, 1984, page 105, abstract no. 155002j, Columbus, Ohio, US; P. HERION et al.: "Monoclonal antibodies against plasma protease inhibitors: II. Production and characterization of 25 monoclonal antibodies against human alpha1-antitrypsin. Correlation between antigenic structure and functional sites", & BIOSCI. REP. 1984, 4(2), 139-47 * Abstract * | 1-4,6-10 | TECHNICAL FIELDS SEARCHED (Int. Cl.4) |

The present search report has been drawn up for all claims

| Place of search | Date of completion of the search | Examiner |
|---|---|---|
| THE HAGUE | 17-07-1989 | VAN BOHEMEN C.G. |